(19) **Europäisches Patentamt / European Patent Office / Office européen des brevets**

(11) **EP 4 352 485 B1**

(12) **EUROPEAN PATENT SPECIFICATION**

(45) Date of publication and mention
of the grant of the patent:
**13.11.2024 Bulletin 2024/46**

(21) Application number: **22773772.3**

(22) Date of filing: **15.09.2022**

(51) International Patent Classification (IPC):
**G01N 1/26** *(2006.01)* **G08B 21/12** *(2006.01)*
**F24F 8/10** *(2021.01)* **A61G 10/02** *(2006.01)*
**F24F 11/30** *(2018.01)* **F24F 11/56** *(2018.01)*
**F24F 11/77** *(2018.01)* **F24F 11/89** *(2018.01)*
**G01N 15/00** *(2024.01)* **G01N 1/22** *(2006.01)*
**F24F 110/64** *(2018.01)* **F24F 110/65** *(2018.01)*

(52) Cooperative Patent Classification (CPC):
**G01N 1/26; F24F 3/16; F24F 11/30; F24F 11/56;**
**F24F 11/77; F24F 11/89; G01N 1/2273;**
**G08B 21/12;** A61G 10/02; B01D 46/442;
B01D 2273/30; F24F 2110/64; F24F 2110/65;
Y02B 30/70

(86) International application number:
**PCT/GB2022/052331**

(87) International publication number:
**WO 2023/041910 (23.03.2023 Gazette 2023/12)**

(54) **AEROSOL CONTROL**

AEROSOLSTEUERUNG

COMMANDE D'AÉROSOL

(84) Designated Contracting States:
**AL AT BE BG CH CY CZ DE DK EE ES FI FR GR**
**HR HU IE IS IT LI LT LU LV MC MK MT NL NO PL**
**PT RO RS SE SI SK SM TR**

(30) Priority: **17.09.2021 GB 202113327**

(43) Date of publication of application:
**17.04.2024 Bulletin 2024/16**

(73) Proprietor: **Air Purity Ltd**
**Newport, CB11 3HZ (GB)**

(72) Inventors:
• **SLOOF, Darren**
**Newport, CB11 3HZ (GB)**
• **PODERICO, Francesco**
**Bicester OX26 2FT (GB)**

(74) Representative: **Potter Clarkson**
**Chapel Quarter**
**Mount Street**
**Nottingham NG1 6HQ (GB)**

(56) References cited:
**WO-A1-2020/146315 CN-A- 102 749 474**
**US-A1- 2022 034 540**

EP 4 352 485 B1

## Description

### Field

The present disclosure relates to an air quality system, an air quality monitoring system and methods of controlling and monitoring air quality.

### Background

Aerosols are known vectors of many diseases. Infectious pathogens can include viruses, bacteria, or fungi, which can spread through breathing, talking, coughing, sneezing, raising of dust, flushing toilets, or any activities which generate aerosol particles or droplets.

Aerosols can pose an infection risk in many indoor settings such as commercial buildings, educational buildings and medical settings. Aerosols can pose a particular infection risk in medical settings such as a hospital where infected patients can shed pathogens that can be transmitted by aerosol and potentially infect hospital staff, visitors and other patients. Medical settings typically have a high footfall providing a large population for disease transmission. Medical settings also typically employ industrial air conditioning and heating systems that can carry aerosols over large distances widening the area of potential transmission. Methicillin-resistant Staphylococcus aureus (MRSA) and COVID-19 are well known examples of pathogens that can spread rapidly within a medical setting via airborne transmission.

It would be desirable to monitor aerosol flow in an indoor setting. It would also be desirable to understand high risk events associated with high levels of aerosol generation. It would further be desirable to control aerosol flow in an indoor setting. The disclosed systems and methods may provide one or more of these desirous effects.

WO2020146315 (A1) describes a system and method for obtaining environmental data namely air quality information from various devices contained within a structure. The various devices contain sensors that can obtain environmental data, which is then analysed by the system to determine if any level of a component within the data is outside of a predefined threshold range.

### Summary

According to a first aspect of the present disclosure there is provided an air quality monitoring system comprising:

  a plurality of particulate matter, PM, sensors, the plurality of PM sensors positioned at a corresponding plurality of positions in a monitoring area;
  and

  an air quality processing device coupled to each of the plurality of PM sensors via a communications network, the air quality processing device configured to:

    receive a particulate level signal from at least two of the plurality of PM sensors; and
    determine particulate matter flow between the at least two PM sensors based on the corresponding particulate level signals.

The air quality monitoring system can advantageously track aerosol flow in a monitoring area. Determining and understanding such aerosol flow in a medical setting can provide a number of advantages including: understanding risk factors associated with aerosol generation; enabling design mitigation measures to reduce aerosol generation and reduce aerosol flow; and enabling activation of intervention measures to reduce the particulate matter and aerosol flow.

The air quality processing device may be configured to determine particulate matter flow between the at least two PM sensors by: detecting an aerosol event at a first of the at least two PM sensors based on the particulate level signal exceeding a first event threshold; and detecting the aerosol event at a second of the at least two PM sensors based on the particulate level signal exceeding a second event threshold.

The first event threshold may comprise an adaptive event threshold.

The second event threshold may comprise a scaled value of the first event threshold.

The air quality processing device may be configured to determine the particular matter flow based on a delay and / or amplitude difference between corresponding peaks of the particulate level signals.

The air quality processing device may be configured to determine the particular matter flow by applying a cross-correlation to the particulate level signals associated with the at least two PM sensors.

The air quality processing device may be configured to identify one or more of: a source of the particular matter flow; a path of the particular matter flow; a velocity of the particular matter flow; an attenuation of the particular matter flow; and / or one or more predicted destinations of the particular matter flow.

The air quality processing device may be further configured to output an intervention signal configured to operate one or more air quality intervention mechanisms.

The air quality intervention mechanisms may comprise one or more of: an automatic door or actuator thereof; operating parameters of an air filtering device; operating parameters of a heating, ventilation and air conditioning, HVAC, system; and an alert signal.

The alert signal may comprise an audible and / or visible alarm signal.

The alert signal may comprise an information signal.

The air quality processing device may be configured to output the intervention signal to operate one or more air quality intervention mechanisms at: a location associated with the source of the particular matter; a location associated with the path of the particular matter flow; and / or a location associated with the one or more potential destinations of the particular matter flow.

The air processing device may be further configured to: analyse the particulate level signal for one or more PM sensors over a time period to determine a particulate matter prevalence associated with the one or more PM sensors; and output prevalence data indicating the particulate matter prevalence.

The prevalence data may indicate: high risk regions of the monitoring area corresponding to one or more PM sensors with a particulate matter prevalence exceeding a first prevalence threshold; and / or low risk regions of the monitoring area corresponding to one or more PM sensors with a particulate matter prevalence less than a second prevalence threshold.

The particulate matter prevalence may include periodic aerosol events associated with the one or more PM sensors. The prevalence data may indicate: the periodic aerosol events; the times of occurrence of the periodic aerosol events; and / or the location of the one or more PM sensors associated with the periodic aerosol event.

The air processing device may be configured to output an intervention signal for operating one or more intervention mechanisms at times corresponding to the periodic aerosol event.

The air processing device may be configured to: receive operational data relating to the monitoring area; correlate one or more aerosol events with the operational data; and identify aerosol event triggers based on the correlation.

Each of the PM sensors may be configured to measure a concentration of particulate matter in air with particle sizes in a range from a lower detection limit to a particulate matter rating of the PM sensor.

Each PM sensor may comprise a plurality of particulate matter ratings and may be configured to measure a plurality of concentrations of particulate matter in air in a corresponding plurality of particle size ranges.

The particulate matter rating may comprise one or more of: 0.5 $\mu$m, 1.0 $\mu$m, 2.5 $\mu$m, 4.0 $\mu$m, 10.0 $\mu$m, 25.0 $\mu$m and 50 $\mu$m.

The lower detection limit may comprise any of: 0.05 $\mu$m, 0.1 $\mu$m, 0.3 $\mu$m and 0.5 $\mu$m.

Two or more of the PM sensors may be positioned at different heights.

The air quality monitoring system may further comprise the communication network.

The air quality monitoring system may further comprise a plurality of further sensors. The further sensors may comprise one or more of: a carbon dioxide (CO2) sensor; a humidity sensor; a temperature sensor; and a pressure sensor.

The air quality monitoring system may comprise a plurality of sensor units each sensor unit comprising: one of the plurality of PM sensors; and one or more further sensors.

According to a second aspect of the present disclosure there is provided a method of monitoring particulate matter flow in a monitoring area, the method comprising:

receiving a plurality of a particulate level signals from at least two particulate matter, PM, sensors positioned in the monitoring area;

determining particulate matter flow between the at least two PM sensors based on the corresponding particulate level signals.

The method may be computer implemented.

According to a third aspect of the present disclosure there is provided an air quality monitoring system comprising:

a plurality of particulate matter, PM, sensors, the plurality of PM sensors positioned at a corresponding plurality of positions in a monitoring area;
and
an air quality processing device coupled to each of the plurality of PM sensors via a communications network, the air quality processing device configured to:

receive a particulate level signal from each of the plurality of PM sensors;
determine an aerosol even based on at least one particulate level signal; and

output an intervention signal configured to operate one or more air quality intervention mechanisms.

According to a fourth aspect of the present disclosure there is provided an air quality monitoring system comprising:

a plurality of particulate matter, PM, sensors, the plurality of PM sensors positioned at a corresponding plurality of positions in a monitoring area;
and
an air quality processing device coupled to each of the plurality of PM sensors via a communications network, the air quality processing device configured to:

receive a particulate level signal from each of the plurality of PM sensors;
analyse the particulate level signal for one or more PM sensors over a time period to determine a particulate matter prevalence associated with the one or more PM sensors; and
output prevalence data indicating the particulate matter prevalence.

According to a fifth aspect of the present disclosure there is provided an air quality system comprising:

a plurality of particulate matter, PM, sensors, the plurality of PM sensors positioned at a corresponding plurality of positions in a monitoring area; and
an air filtering device wirelessly coupled to the at least one PM sensor, the air filtering device configured to:

receive a particulate level signal from one or more of the plurality of PM sensors; and
adjust a fan speed of the air filtering device in response to the particulate level signal.

Adjusting the fan speed in response to the particulate level signal from the PM sensors can advantageously provide on-demand, selective control of the air filtering device.

The air filtering device may be further configured to adjust the fan speed based on a distance between each of one or more of the plurality of PM sensors and the air filtering device.

The air filtering device may be configured to determine the distance between each of the one or more of the plurality of PM sensors and the air filtering device based on a received signal strength indicator of the particulate level signal.

Each of the PM sensors may be configured to measure a concentration of particulate matter in air with particle sizes in a range from a lower detection limit to a particulate matter rating of the PM sensor.

Each PM sensor may comprise a plurality of particulate matter ratings and may be configured to measure a plurality of concentrations of particulate matter in air in a corresponding plurality of particle size ranges.

The particulate matter rating may comprise one or more of: 0.5 $\mu$m, 1.0 $\mu$m, 2.5 $\mu$m, 4.0 $\mu$m, 10.0 $\mu$m, 25.0 $\mu$m and 50 $\mu$m.

The lower detection limit may comprise any of: 0.05 $\mu$m, 0.1 $\mu$m, 0.3 $\mu$m and 0.5 $\mu$m.

Each of the plurality of PM sensors may be configured to measure a concentration of particulate matter in air for a plurality of particle sizes. The air filtering device may be configured to adjust the fan speed based on: the distance between each of the one or more PM sensors of the plurality of PM sensors and the air filtering device; and the concentration of particulate matter for each of the plurality of particle size ranges for each of the one or more PM sensors of the plurality of PM sensors.

The air quality system may further comprise a server, wherein the plurality of PM sensors and / or the air filtering device are communicatively coupled to the server over a communications network.

The air quality system may further comprise a plurality of further sensors. The, further sensors may comprise one or more of: a carbon dioxide ($CO_2$) sensor; a humidity sensor; a temperature sensor; and a pressure sensor.

The air quality system may comprise a plurality of sensor units each sensor unit comprising: one of the plurality of PM sensors; and one or more further sensors.

The air filtering device may comprise any of: a ventilation system; a heating, ventilation and air conditioning, HVAC, system; and an air purifier.

The air filtering device may comprise an air purifier comprising one or more of: a high-efficiency particulate air, HEPA, filter; a carbon filter; and a UVC lamp.

Two or more of the PM sensors may be positioned at different heights.

There may be provided a computer program, which when run on a computer, causes the computer to configure any apparatus, including a circuit, controller, converter, or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

Brief Description of the Drawings

One or more embodiments will now be described by way of example only with reference to the accompanying drawings in which:

Figure 1 illustrates an air quality system for controlling aerosol flow in a medical setting according to an embodiment of the present disclosure;
Figure 2 illustrates an air quality monitoring system according to an embodiment of the present disclosure;
Figure 3 illustrates another air quality monitoring system according to an embodiment of the present disclosure;
Figure 4 illustrates particulate matter sensor data for an aerosol event captured by the air quality monitoring system of Figure 3;
Figure 5 illustrates carbon dioxide sensor data for the aerosol event captured by the air quality monitoring system of Figure 3;
Figures 6A and 6B show cross correlations between particulate matter sensor data for the captured aerosol event for a pair of sensors of Figure 3;
Figures 7A and 7B show cross correlations between particulate matter sensor data for the captured aerosol event for another pair of sensors of Figure 3;
Figures 8A and 8B show cross correlations between particulate matter sensor data for the captured aerosol event for a further pair of sensors of Figure 3;
Figures 9A and 9B show cross correlations between particulate matter sensor data for the captured aerosol event for a yet further pair of sensors of Figure 3; and
Figures 10A to 10D show cross correlations between particulate matter sensor data and CO2 sensor data for various pairs of sensors of Figure 3.

Detailed Description

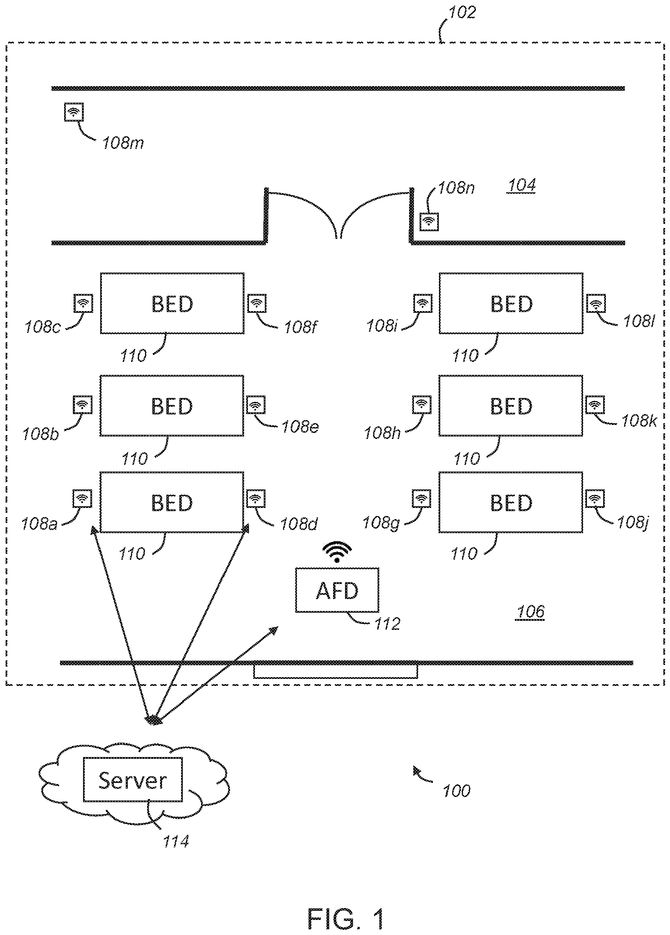

Figure 1 illustrates an air quality system 100 for controlling aerosol flow in an indoor setting according to an embodiment of the present disclosure. In this example, the indoor setting is a medical setting 102 comprising a portion of a hospital ward including a corridor 104 and a ward bay 106. The air quality system 100 comprises a plurality of particulate matter (PM) sensors 108a..108n. In this example, the air quality system comprises 14 PM sensors 108a..108n placed at a corresponding plurality of positions in the medical setting 102. The 14 PM sensors include 12 PM sensors 108a..108l in the ward bay 106, with two PM sensors adjacent to each bed 110, and 2 PM sensors in the corridor. The air quality system 100 further comprises an air filtering device 112 wirelessly coupled to the PM sensors 108a..n (referred to collectively as PM sensors 108) . The air filtering device 112 receives a particulate level signal from each of the sensors 108a-108n and adjusts a fan speed of the air filtering device 112 in response to the particulate level signal. The air filtering device 112 is placed at a position separated from the plurality of positions of the PM sensors 108. In other words the PM sensors 108 are remote to the air filtering device 112.

As disclosed herein, an air filtering device 112 may comprise any device capable of reducing the PM content in the air. For example, the air filtering device 112 may comprise a ventilation system, a heating, ventilation and air conditioning (HVAC) system (such as those installed in commercial settings and hospitals), or a stand-alone air purifier. The air filtering device 112 includes a fan for drawing air through the device. An increase in fan speed provides an increase in the rate of air purification / PM reduction.

Adjusting the fan speed in response to the particulate level signal from the PM sensors 108 can advantageously provide on-demand, selective control of the air filtering device 112. For example, the air filtering device 112 can increase the fan speed to provide a high rate of PM reduction when the PM sensor 108 indicates a relatively high PM content and reduce the fan speed to reduce the rate of PM reduction when the PM sensor indicates a relatively low PM content. As a result, the air filtering device 112 can operate with reduced energy consumption and noise pollution while still maintaining sufficient PM reduction during periods of relatively high PM content in the air.

Furthermore, by monitoring the medical setting 102 with PM sensors 108, the system 100 can advantageously monitor particles directly associated with infectious vectors. For example, MRSA is known to transmit via dead skin which can constitute particulate matter sizes on the order of 25 - 50 $\mu$m. Airborne viruses, such as COVID-19 have been shown to associate with (inhalable) particulate matter sizes less than 2.5 $\mu$m. Infectious aerosols and particulate matter flow may be generated directly by a patient breathing, sneezing, coughing etc or indirectly by the perturbation of items containing infectious particulates such as the flapping of bedsheets, the drawing of curtains etc. PM sensors 108 can advantageously detect all sources of particular matter flow in contrast to CO2 sensors which may only detect regions of stagnant air / poor ventilation or the presence of a large number of people.

As disclosed herein, particulate matter, particulate matter sensors and particular matter sensor ratings are referred to as understood in the art. Particulate matter may refer to a mixture of solid particles and liquid droplets found in the air. Particulate matter may refer to particle sizes greater than 0.1 $\mu$m, for example 0.1 $\mu$m to 50 $\mu$m. Particulate matter does not refer to individual molecules such as molecules of CO2.

The PM sensors 108 may measure a concentration (micrograms per cubic metre) of particulate matter in air for a range of particle sizes. A PM sensor may measure a concentration of particulate matter for particles sizes between a lower detection limit and a particulate matter rating. The lower detection limit may be on the order of 0.1 $\mu$m, for example 0.05 $\mu$m, 0.1 $\mu$m, 0.3 $\mu$m or 0.5 $\mu$m.

A particulate matter rating may refer to the upper limit of particle size of the measurement range. As an example, a PM sensor 108 with a particulate matter rating of 2.5 $\mu$m (PM 2.5) may measure a concentration of particulate matter with particle sizes in a range from the lower detection limit to 2.5 $\mu$m. The PM sensors 108 may comprise a particulate matter rating of any of: 0.5 $\mu$m (PM0.5), 1.0 $\mu$m (PM1), 2.5 $\mu$m, (PM2.5), 4.0 $\mu$m (PM4), 10.0 $\mu$m (PM10), 25.0 $\mu$m (PM25) and 50.0 $\mu$m (PM50). The PM sensors may comprise multiple particulate matter ratings corresponding to multiple particle size ranges. The particulate level signal for each sensor may comprise concentration values for each of the one or more particle size ranges. Particulate matter with particle sizes less than 10 $\mu$m may referred to as inhalable and particle sizes less than 2.5 $\mu$m may be referred to as fine inhalable. Monitoring these particle sizes advantageously directly monitors particulate matter associated with airborne disease transmission.

The PM sensors 108 may be coupled to the air filtering device 112 via a local wireless connection such as via a WiFi network, a Bluetooth (classic or Bluetooth low energy) connection or other known local wireless connections. The PM sensors 108 may each communicate directly with the air filtering device 112 (rather than via a communications network).

In this example, the air filtering device 112 comprises an air purifier positioned in the ward bay 106. The air purifier can comprise one or more filtering mechanisms. The one or more filtering mechanisms may include any of: a high-efficiency particulate air (HEPA) filter, a carbon filter and a UVC lamp.

The air filtering device 112 may adjust a fan speed based on the particulate level signal from each PM sensor 108 and a distance between the PM sensor 108 and the air filtering device. In this way, the system 100 can account for the effectiveness of the PM reduction of the air filtering device 112 at the position of the relevant PM sensor 108. In some examples, the fan speed may be based on the square of the distance to each PM sensor 108. For example, a function for determining the fan speed may take the form:

$$Speed_{fan} = f\left(\sum_{i}^{n} k_i x_i^2\right)$$

where i is a PM sensor index, n is the total number of sensors, $k_i$ is a calibration constant for the $i^{th}$ sensor and $x_i$ is the distance between the $i^{th}$ sensor and the air filtering device 112.

In some examples, the air filtering device 112 may adjust the fan speed based on the distance to the each of the PM sensors 108 and the corresponding concentration values for each of a plurality of particle sizes. In one example, a function for determining the fan speed may take the form:

$$Speed_{fan} = \sum_{i}^{n} f_1(x_i PM1) + f_2(x_i PM2.5) + f_3(x_i PM4) + f_4(x_i PM10)$$

In some examples, the functions f1..f4 may take the form:

$$f_1 = k_{11} x_i PM1 + k_{12} x_i^2 PM1$$

$$f_2 = k_{21}x_i PM2.5 + k_{22}x_i^2 PM1$$

$$f_1 = k_{31}x_i PM4 + k_{32}x_i^2 PM1$$

$$f_1 = k_{41}x_i PM10 + k_{42}x_i^2 PM1$$

where $k_{11}$, $k_{12}$, $k_{21}$, $k_{22}$, $k_{31}$, $k_{32}$, $k_{41}$, $k_{42}$, are either constants or derived by a look up table or index match.

In some examples, the PM sensors 108 may be fixedly positioned in predetermined locations such that the air filtering device 112 can store the distance to each sensor 108 in a memory. In other examples, the PM sensors 108 may be positionable by a user. As a result, the air filtering device 112 may determine the distance to a PM sensor 108 based on a received signal strength indicator (RSSI) of the particulate level signal. In this way, users can advantageously re-position the PM sensors 108 as desired. For example, in a hospital setting sensors may be repositioned to protect vulnerable patients.

In some examples, two or more PM sensors 108 may be positioned at different heights. The two or more sensors may be located either side of a monitored object or area such as either side of a bed 110 or either side of the ward bay 106. Positioning two sensors at different heights can indicate a decay or fall rate of an aerosol. The fall rate may be associated with (higher concentrations of) larger particulate size. Understanding suspension level of aerosols at different heights can indicate a potential particulate matter travel or flow and associated risks. The different height sensors may also indicate aerosol events from one side of the room and / or how an aerosol may move from the foot of the bed to above the patient head. The different height sensors can track the aerosol flow using a time lapse and indicate a speed of potentially infectious aerosols. The time lapse may indicate an air change rate deliverable requirement of the air filtering unit 112 and / or an optimal position for an inlet of the air filtering unit to reduce or prevent aerosol migration across the monitoring area 102.

In some examples, the system 100 may comprise one or more further sensors. The one or more further sensors may comprise a plurality of further sensors located at a plurality of positions in the monitoring area 102. In some examples, one or more further sensors may be co-located with each of the PM sensors 108. For example, each of the PM sensors 108 may form part of a sensor unit which comprises one or more further sensors. The one or more further sensors may comprise one or more of: a carbon dioxide (CO2) sensor, a humidity sensor, a temperature sensor and a pressure sensor. The air filtering device 112 may adjust a fan speed based on one or more further signals corresponding to the one or more further sensors. The one or more further sensors may provide additional data to support the system 100. A temperature and humidity sensor can represent a controlled environment provided by an HVAC system, which should be stable throughout a ward bay 106 to minimise potential convection currents and a resulting faster potential spread of anything infectious. A pressure sensor can be helpful for monitoring air pressure dynamics which can influence air flow and may change in response to operation of the air filtering unit 112. A CO2 sensor can monitor CO2 concentrations which can correlate with particulate matter PM1 and lower. Therefore, a CO2 sensor can provide an additional indication of increased risks and activity of people. For example, at peak times of the day particle levels should remain low due to the performance of the air filtering device 112, however CO2 level may rise which can be associated with any increase in particulate levels. The system 100 may output a signal indicative of measurements of the one or more further sensors. The output signal may include an alert that a temperature, humidity or pressure gradient has been detected. In some examples, the output signal may be provided to an HVAC system to rectify a temperature, humidity or pressure gradient.

In some examples, the air filtering device may comprise a PM sensor and / or one or more further sensors.

In some examples, the system 100 may comprise one or more outdoor sensors, for example sensors positioned outside a hospital building. The one or more outdoor sensors may include one or more of the PM sensors and/or one or more of the further sensors. The outdoor sensors may monitor humidity, wind currents, temperatures, PM counts, pressure etc. The outdoor sensors allow the system 100 to account for seasonal variation in particulate levels. For example, a temperature gradient between the outside and inside of the building can create air flows that carry particulate matter. As a further example, background particulate levels may vary seasonally due to a variation in airborne pollen.

In some examples, the system 100 may optionally comprise a server 114. The server 114 may be positioned in the medical setting 102 or may be located elsewhere (in the cloud). The sensors 108 and / or the air filtering device 112 may comprise a transceiver enabling communication with the server 114 over a communications network, such as a local area network or the internet. The sensors 108 and / or the air filtering device 112 may output the particulate level signals to the server 114 for storage, monitoring, analysis and / or intervention. A second aspect of the disclosure, described below, relates to a monitoring system comprising a plurality of PM sensors connected to an external air quality processing device over a communications network. It will be appreciated that the functionality described in relation to the second aspect may apply equally to the first aspect described in relation to Figure 1.

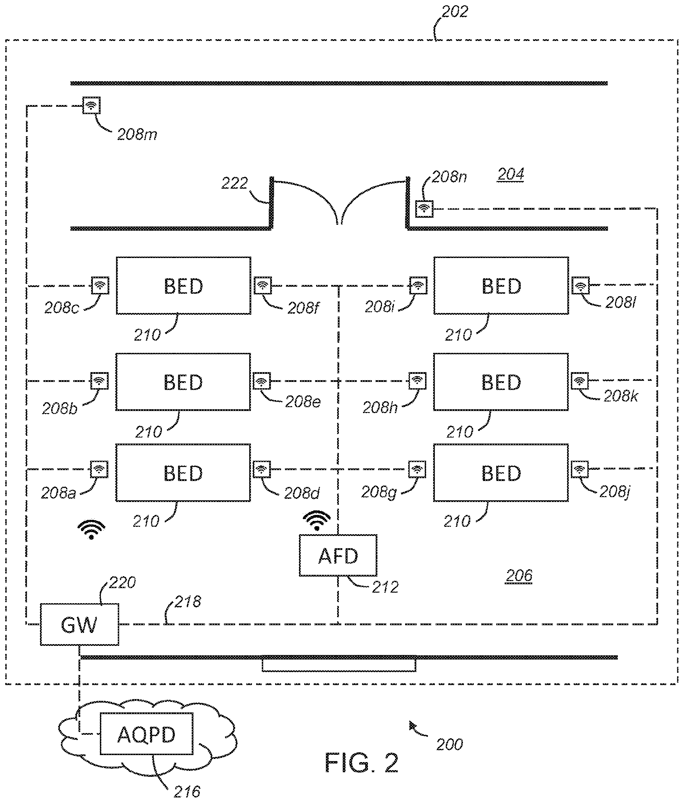

Figure 2 illustrates an air quality monitoring system 200 according to an embodiment of the present disclosure. Features of Figure 2 that are also present in Figure 1 have been given corresponding reference numbers in the 200 series and will not necessarily be described again here.

The air quality monitoring system 200 is arranged for monitoring aerosol flow in an indoor setting 202. In this example, the indoor setting comprises a medical setting 202 comprising a portion of a hospital ward including a corridor 204 and a ward bay 206. The air quality monitoring system 200 comprises a plurality of PM sensors 208a..208n positioned at a corresponding plurality of positions in the medical setting 202. Each of the plurality of sensors 208 is coupled to a communications network 218 . The air quality monitoring system 200 further comprises an air quality processing device 216 coupled to each of the plurality of PM sensors by the communications network 218. The air quality processing device 218 may comprise one or more processors located on a back-end server. The back-end server may be located in another part of the medical setting or remotely to the medical setting 102 such as in the cloud. The air quality processing device 218 may be configured to receive a particulate level signal from at least two of the plurality of PM sensors 208. The air quality processing device 218 can process the particulate level signals and determine particulate matter flow between the at least two of the PM sensors 208 based on the corresponding particulate level signals.

The air quality monitoring system 200 can advantageously track aerosol flow in a monitoring area. Determining and understanding such aerosol flow in a medical setting can provide a number of advantages including: understanding risk factors associated with aerosol generation; enabling design mitigation measures to reduce aerosol generation and reduce aerosol flow; and enabling activation of intervention measures to reduce the particulate matter and aerosol flow.

It will be appreciated, that any of the features of the system 100 (such as the further sensors and fan speed adjustment) and any of the features of the PM sensors 108 (such as the particle size ranges, advantages of monitoring particular particle size ranges, and positioning of PM sensors) described above in relation to Figure 1, may equally apply to the system 200 and PM sensors 208 of Figure 2, and vice versa.

The communications network 218 may comprise a local area network and / or a wide area network such as the internet. The communications network 218 may include wired and / or wireless communication paths. The communications network 218 may also include a local gateway 220 (or hub) for communicating: (i) locally with the plurality of PM sensors 208, optionally over a wireless network such as WiFi; and (ii) over a wide area network with the air quality processing device 218. In other examples the PM sensors 208 may communicate directly with the air processing device 216 via a wired network or over a wireless network such as a mobile communications network or a WiFi network.

The air quality monitoring system 200 may determine the particulate matter flow between two or more PM sensors 208 based on a delay and / or amplitude difference between corresponding peaks in the particulate level signals. In some examples, the air quality processing device 216 may determine the particulate matter flow between two PM sensors by: (i) detecting an aerosol event at a first PM sensor based on the particulate level signal for the first PM sensor exceeding a first event threshold; and (ii) detecting the same aerosol event at a second PM sensor based on the particulate level signal for the second PM sensor exceeding a second event threshold. The second event threshold may be less than or equal to the first event threshold.

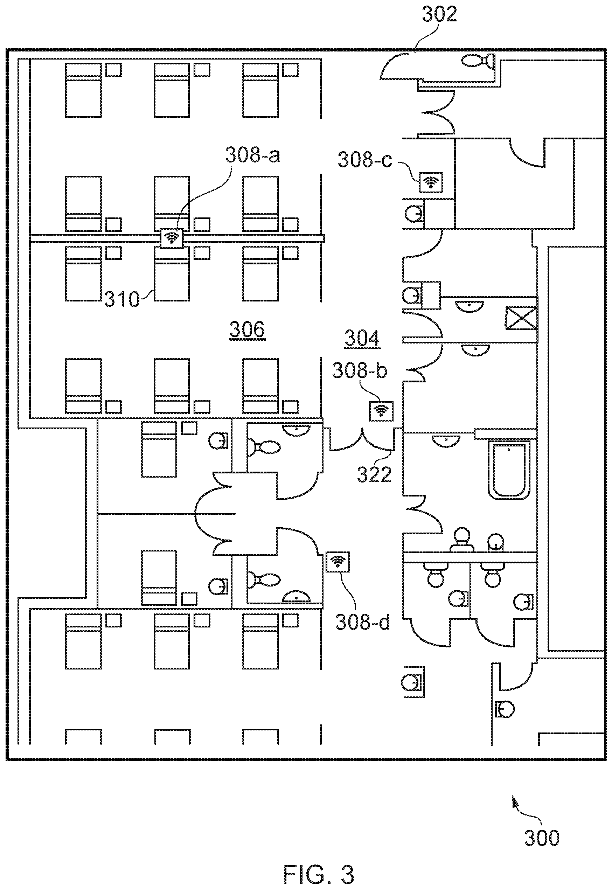

As an example, if an aerosol (particulate matter) generating event (referred to as aerosol event) such as a patient sneezing or flapping their bedding occurs at a bed 210 adjacent to PM sensors 208h, 208k, the air quality processing device 216 may detect a peak on a first PM sensor 208h adjacent to the bed 210 based on the particulate level signal exceeding a first event threshold. At a later time, the air quality processing device 216 may detect a peak corresponding to the same aerosol event at one or more of the remaining sensors 208a..208g, 208i..208l in the ward bay 206 based on a corresponding particulate level signal exceeding the second event threshold. At a yet later time, the air quality processing device 216 may detect a peak corresponding to the same aerosol event on a further PM sensor 208m located in the corridor 204 based on a corresponding particulate level signal exceeding the second event threshold. In this way, the air quality monitoring system 200 can track aerosol flow from the aerosol event in the medical setting 202. Further discussion of example data illustrating the tracking of an aerosol generation event is described below in relation to Figures 3 to 10.

The first and second event thresholds enable the monitoring system 200 to detect aerosol events as peaks in the particulate level signal above the expected background level. The first and / or second event thresholds may comprise an adaptive event level threshold with a value that adapts according to a changing background level of particulate matter. For example, in a hospital setting, a higher background level would be expected during the day compared to night and a yet higher background level would be expected during visiting hours or ward rounds etc. The first and / or second event thresholds may comprise time-dependent adaptive threshold that change according to a time of day and / or particular day (weekend versus weekday). The threshold levels may be determined following an initial calibration period following installation of the system 200.

The air processing device 216 may identify one or more parameters associated with the particulate matter flow between the two or more PM sensors 208. The one or more parameters may include any of: (i) a source of the aerosol flow based on a position of the first PM sensor 208 to detect the aerosol event; (ii) a path, or direction of travel, of the particulate matter flow based on vectors connecting the two or more PM sensors 208 detecting the aerosol event and, optionally, the attenuation between the particle signal level of the first PM sensor to detect the aerosol event and each subsequent sensor to detect the aerosol event; (iii) a velocity of the aerosol flow based on a delay time between corresponding peaks in the two or more PM sensors 208 detecting the aerosol event; (iv) an attenuation of the aerosol flow based on the reduction of the particle signal level of the first PM sensor to detect the aerosol event and each subsequent sensor to detect the aerosol event; and (v) one or more potential destinations of the aerosol flow based on the path of the aerosol flow, the attenuation of the aerosol flow and / or the velocity of the aerosol flow.

In some examples, the air quality monitoring system 200 may comprise one or more further sensors. The one or more further sensors may comprise a plurality of further sensors located at a plurality of positions in the monitoring area 202. In some examples, one or more further sensors may be co-located with each of the PM sensors 208. For example, each of the PM sensors 208 may form part of a sensor unit which comprises one or more further sensors. The one or more further sensors may comprise one or more of: a carbon dioxide (CO2) sensor, a humidity sensor, a temperature sensor and a pressure sensor. As discussed above in relation to the first embodiment and further below, monitoring temperature, humidity and / or pressure can help: (i) identify factors contributing to a detected aerosol event and flow; and (ii) identify intervention measures for mitigating the aerosol flow. Monitoring CO2 can help identify: (i) regions of stagnant air / poor ventilation; and (ii) a source of an aerosol event as arising from human respiratory activity versus mechanical activity (opening of curtains, flapping of bed-sheets etc).

In some examples, the system 200 may comprise one or more outdoor sensors, for example sensors positioned outside a hospital building. The one or more outdoor sensors may include one or more of the PM sensors and/or one or more of the further sensors. The outdoor sensors may monitor humidity, wind currents, temperatures, PM counts, pressure etc. The outdoor sensors allow the system 200 to account for seasonal variation in particulate levels. For example, a temperature gradient between the outside and inside of the building can create air flows that carry particulate matter. As a further example, background particulate levels may vary seasonally due to a variation in airborne pollen.

In some examples, the air quality processing device 216 may output an intervention signal in response to determining a particulate flow between two or more PM sensors 208. The air quality processing device 216 may output the intervention signal via the communications network 218 to one or more (network connected) air quality intervention mechanisms 222. In some examples, the air quality monitoring system 200 may include the one or more air quality intervention mechanisms 222. In the example of Figure 2, the one or more air quality intervention mechanisms includes automatic doors 222 on the ward bay 206.

The one or more air quality intervention mechanisms may include one or more of: an automatic door or actuator thereof; operating parameters of an air filtering device; operating parameters of a heating, ventilation and air conditioning, HVAC, system; and an alert signal. The air quality system may output the intervention signal to activate (or operate) one or more air quality intervention mechanisms at: a location associated with the source of the particulate matter flow (aerosol event); a location associated with the path of the particular matter flow; and / or a location associated with the one or more potential destinations of the particular matter flow.

In some examples, the air quality system 200 may output the intervention signal to activate one or more automatic doors or other isolation means to isolate a particular area associated with the particulate matter flow. In this way, the air quality monitoring system 200 can isolate a particulate matter flow to a restricted area and reduce the risk of airborne transmission of infectious particles.

In some examples, the air quality system 200 may output the intervention signal to activate, or adjust the operating parameters (eg fan speed) of, an air filtering device 212 to increase filtering of the air and reduce the particulate matter content. The system 200 may output the intervention signal to one or more air filtering devices 212 at a source or along a path of the particulate matter flow. In this way, the detected particulate matter can be reduced thereby reducing its further spread. The system 200 may output the intervention signal to one or more air filtering devices 212 at one or more predicted destinations of the particulate matter flow. In this way, the system 200 can take preventative action to maximise air filtering in an area before the particulate matter flow arrives. The air filtering device 212 may comprise any of: a ventilation system; an HVAC, system; and an air purifier. The air purifier may comprise one or more of: a HEPA, filter; a carbon filter; and a UVC lamp.

In some examples, the air quality system 200 may output the intervention signal to activate, or adjust the operating parameters of, an HVAC system. For example, the system 200 may output the intervention signal to adjust a temperature, ventilation or humidity of an area associated with one or more of the PM sensors. In some examples, the system 200 may comprise a temperature, humidity, pressure and / or CO2 sensor co-located with each PM sensor 208. In this way, the system 200 can determine regions associated with the PM flow having a temperature, humidity, pressure and / or CO2 level, or gradients thereof, above a corresponding threshold level. The intervention signal may adjust a HVAC system to reduce regions of high temperature, humidity, pressure or CO2 level (or gradients thereof) accordingly.

In some examples, the air quality system 200 may output the intervention signal to activate an alert. The alert may include an audible and / or visible alarm signal such as a siren or flashing light. The alert may comprise an information signal, such as a warning message or graphic on a computer system or an email, text message, push notification or other alert mechanism as known in the art. The information signal may comprise a graphical representation of the indoor setting to indicate the source, path and / or potential destinations of the PM flow. The graphical representation may be colour coded to indicate a magnitude (and risk level) of the PM flow. The alert signal may alert one or more users (such as hospital staff) to an aerosol event and the user can investigate the source of the alert and / or take remedial action.

In some examples, the air quality monitoring system 200 may comprise a memory for storing the particulate level signal from each of the one or more PM sensors 208. The memory may also store data received from the one or more further sensors.

The air processing device 216 may analyse the particulate level signal for one or more PM sensors 208 over a time period (for example, an hour, a day, a week or a month) to determine a particulate matter prevalence in an area associated with the one or more PM sensors 208. The particulate matter prevalence may relate to a number, or frequency, of particulate matter flows or aerosol events associated with the one or more PM sensors 208. The particular matter prevalence may relate to an average particulate level signal, a total period of time spent above an event threshold or any other suitable particulate level signal metric for the one or more PM sensors 208.

By determining the particulate matter prevalence for one or more PM sensors 208, the air quality monitoring system 200 may determine high-risk areas and low-risk areas of the medical setting 202 associated with respectively relative high or low levels of aerosol events and / or particulate matter flows. For example, the air processing device 212 may determine high-risk areas of the medical setting 202 as areas with a particulate matter prevalence exceeding a first prevalence threshold and similarly determine low-risk areas of the medical setting as areas with a particulate matter prevalence less than a second prevalence threshold. The air processing device 216 may output data, such as reports, graphics etc, indicating the high-risk areas and low-risk areas. As a result, a user can redesign an indoor setting accordingly. For example, low-risk areas (for example a bed in an alcove or next to an air filtering device 212) may be designated for placing high risk, infectious patients such that there is minimal particulate matter flow to transmit their infection. Correspondingly, high-risk areas associated with aerosol generation events may be designated as only suitable for low-risk non-infectious patients. As a further example, additional intervention mechanisms could be identified for reducing particulate matter flow in high-risk areas.

In some examples, the air processing device 212 may analyse the particulate level signal for one or more PM sensors 208 over a time period to determine periodic aerosol events associated with the one or more PM sensors 208. For example, the air processing device 212 may determine a regular occurrence (daily, weekly etc) of the same particulate matter flow and / or aerosol event. Such periodic events may be generated by routine activities, which in a hospital setting may be any of a ward round, visiting time, meal time, the opening of curtains or other regular events.

In some examples, the air processing device 212 may output data indicating the periodic aerosol event, the times of occurrence of the periodic aerosol event and / or the position of the one or more PM sensors associated with the periodic aerosol event. In this way, a user may correlate the periodic aerosol event with a periodic activity or event and take appropriate remedial measures, such as redesigning the area, preventing the periodic activity, or adjusting the process of the periodic activity to minimise aerosol events (for example reducing the number of staff on a ward round).

In some examples, the air processing device 212 may output an intervention signal for operating one or more intervention mechanisms, in the vicinity of the one or more PM sensors 208 associated with the periodic aerosol event, at times corresponding to the periodic aerosol event. The air processing device 212 may output the intervention signal to the one or more intervention mechanisms shortly before an occurrence of the periodic aerosol event. For example, the air processing device may increase the fan speed of an air filtering device and / or activate an automatic door to isolate the area.

In some examples, the air processing device 212 may receive operational data associated with the monitoring area 202. The air processing device 212 may receive the operational data from a computer system associated with the monitoring area, manual user input and / or from sensor input. The operational data may include details of operational events or clinical events such as ward rounds, meal times, visiting times, patient admission and discharge, patient and staff illness etc. The air processing device 212 may correlate the operational detail with an aerosol event, a periodic aerosol event or a particulate matter flow to identify aerosol event triggers such that a root cause of the event can be identified. A user and / or the air quality monitoring system 200 can then take remedial action and / or implement mitigating interventions as described above. In some examples, the air processing device 212 may implement an algorithm to correlate the operational detail with aerosol events. The algorithm may be an artificial intelligence (AI) algorithm.

The air processing device 212 / algorithm may perform some pre-processing on the particulate level signals prior to identifying aerosol events, periodic aerosol events or particulate matter flows. The air processing device 212 / algorithm may perform one or more of the following functions:

- Noise removal
- Scaling
- Normalization - During a calibration period, the air processing device may define normal values for the particulate level signals on a day base / week base etc. In this way, the algorithm may determine the first and / or second event thresholds based on the normal values.

- Vectorization - The air processing device 212 may determine a particulate matter flow based on a peak of a first particulate level signal exceeding a first event threshold at a first PM sensor at a first time and a corresponding peak of a second particulate level signal exceeding a second event threshold at a second PM sensor at a second time (shortly) after the first time. The air processing device 212 may define a "vector" of particulate matter flow from the fist PM sensor to the second PM sensor.
- Periodical Normalization - The air processing device 212 may identify periodic anomalies / peaks that exceed the first event threshold to determine a periodic aerosol event
- Decision tree/ Heuristic search/ specific algorithm - the air processing device 212 may perform one or more actions to mitigate aerosol events, periodic aerosol events and / or particulate matter flow, including:

  ◦ Outputting an intervention signal to remotely control one or more intervention mechanisms such as activating automatic doors or increasing the fan speed in an air purifier following a detected aerosol event or at times corresponding to the periodic aerosol event;
  ◦ Output information to a user to take remedial action. The user may be an operator who can adjust the settings on an intervention mechanism such that it activates or increases effectiveness at times corresponding to the periodic aerosol event.

Although the embodiment of Figure 2 is described in relation to determining particulate matter flow between two sensors, it will be appreciated that determining particulate matter flow is optional. In some examples, the system 200 may implement any of the interventions or data analysis and output described above, based on the detection of an aerosol event as a particulate level signal of one or more PM sensors 208 exceeding the first event threshold.

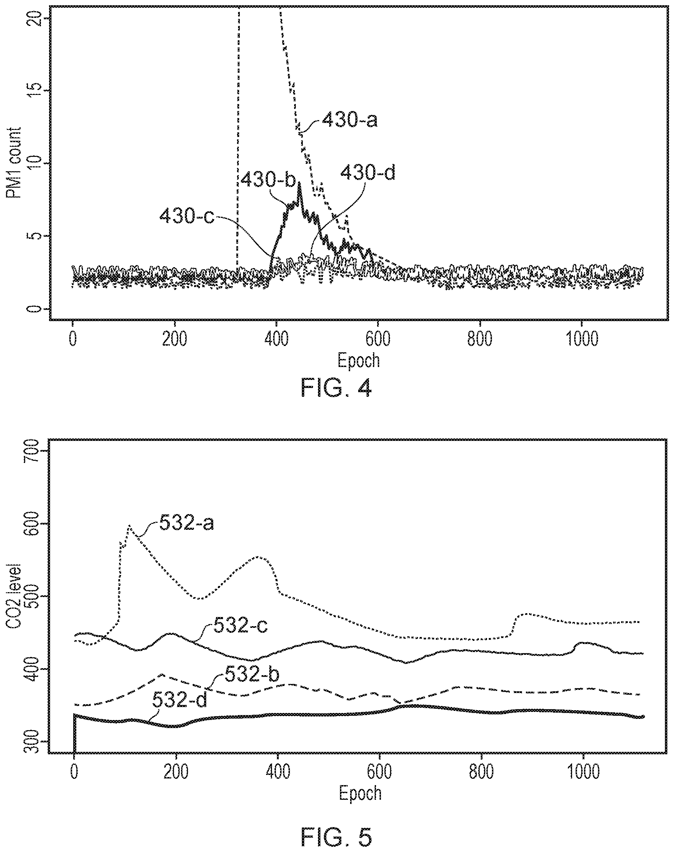

Figure 3 illustrates another air quality monitoring system 300 according to an embodiment of the present disclosure and Figures 4 to 10d illustrate PM sensor data and analysis for an aerosol event captured by the air quality monitoring system 300. Features of Figure 3 that are also present in Figure 1 or Figure 2 have been given corresponding reference numbers in the 300 series and will not necessarily be described again here.

The air quality monitoring system 300 includes a plurality of PM sensors 308-a, 30b-b, 308-c and 308-d situated in a medical setting 302. A first PM sensor 308-a is situated in a ward bay 306. A second PM sensor 308-b is situated in a corridor 304 and adjacent to the ward bay 306. A third PM sensor 308-c is situated in the corridor 304 in an alcove along from the ward bay 304. A fourth PM sensor 308-d is situated in the corridor 304 on the other side of corridor doors 322 from the second PM sensor 308-b. The system 300 otherwise has the same structural and functional features as the system of Figure 2, including an air processing device (not illustrated) and its associated functionality.

An aerosol event occurred at bed 310 when a patient fell out of the bed 310 at night when they were asleep.

Figure 4 shows particulate level signals 430-a, 430-b, 430-c, 430-d corresponding to PM1 concentration (particle sizes between lower detection limit and 1 $\mu$m) at each of the PM sensors 308 in the time period surrounding the aerosol event. The horizontal axis corresponds to time in epochs wherein each epoch is equal to 5 seconds.

A first particulate level signal 430-a corresponds to the first PM sensor 308-a in the ward bay 306 and shows a large peak (off the vertical scale) corresponding to the aerosol event. The second particulate level signal 430-b corresponds to the second PM sensor 308-b in the corridor 304 and shows a peak delayed from the peak of the first particulate level signal 430-a by about 5 minutes. The delay corresponds to the time that the particulate matter flow took to travel from the bed 310 to the second PM sensor 308-b. The third particulate level signal 430-c corresponds to the third PM sensor 308-c in the alcove. A shallow peak is just visible around the same time as the peak in the second particulate level signal 430-b. The fourth particulate level signal 430-d corresponds to the fourth PM sensor 308-d in the alcove. A shallow peak is just visible around the same time as the peak in the second particulate level signal 430-b.

Figure 5 shows CO2 level signals 532-a, 532-b, 532-c, 532-d corresponding to CO2 concentration at each of the PM sensors 308 (system 300 also includes CO2 sensors co-located with PM sensors 308) in the time period surrounding the aerosol event. A peak in CO2 level can be seen in a first CO2 level signal 532-a corresponding to a CO2 sensor co-located with the first PM sensor 308-a. The rise in CO2 may be associated with the aerosol event. No corresponding peak can be seen in the CO2 level signals 532-b, 532-c, 532-d corresponding to CO2 sensors co-located with the PM sensors 308-b, 308-c, 308-d in the corridor 304. The data illustrates that aerosol flow cannot be determined or tracked using CO2 sensors alone.

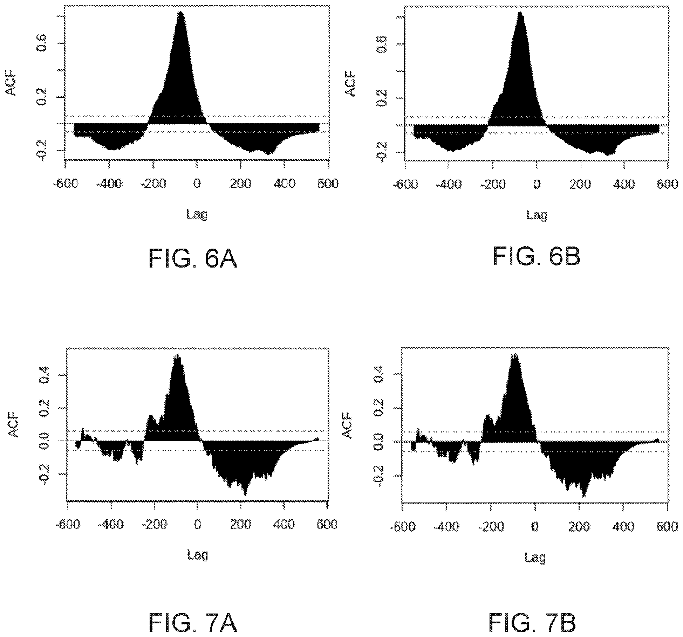

As outlined above, the air processing device of the system 312 may determine a particulate matter flow between at least two PM sensors 308. One approach to this is to apply a cross-correlation between the relevant particulate level signals. This can help identify peaks in different particulate level signals that may not be visible to the naked eye (e.g. the peaks in the third and fourth particulate level signals in Figure 4 are difficult to discern). Figures 6a to 9b show various cross-correlations between particulate level signals 430a-430d from the PM sensors 308a-308d in the time period surrounding the aerosol event.

Figure 6A shows the cross-correlation between a first PM1 particulate level signal from the first PM sensor 308-a and a second PM1 particulate level signal from the second PM sensor 308-b. A correlation peak (max r = 0.836) is seen at a time lag of 74 epochs (370 seconds). This indicates that PM1 particles are travelling from the ward bay 306 into the corridor 304.

Figure 6B shows the cross-correlation between a first PM10 particulate level signal from the first PM sensor 308-a and a second PM10 particulate level signal from the second PM sensor 308-b. A correlation peak (max r = 0.836) is seen at a time lag of 74 epochs (370 seconds). This indicates that PM10 particles are travelling from the ward bay 306 into the corridor 304.

Figure 7A shows the cross-correlation between a first PM1 particulate level signal from the first PM sensor 308-a and a fourth PM1 particulate level signal from the fourth PM sensor 308-d. A correlation peak (max r = 0.527) is seen at a time lag of 92 epochs (460 seconds). This indicates that PM1 particles are travelling from the ward bay 306 into the corridor 304 and then travelling along the corridor 304. The correlation is not as strong as in Figure 6A indicating attenuation of the particulate matter flow as it proceeds along the corridor 304. The time lag is also greater reflecting the additional time to travel along the corridor 304.

Figure 7B shows the cross-correlation between a first PM10 particulate level signal from the first PM sensor 308-a and a fourth PM10 particulate level signal from the fourth PM sensor 308-d. A correlation peak (max r = 0.522) is seen at a time lag of 92 epochs (460 seconds). This indicates that PM10 particles are travelling from the ward bay 306 into the corridor 304 and then travelling along the corridor 304. The correlation is not as strong as in Figure 6A indicating attenuation of the particulate matter level as it travels along the corridor 304. The time lag is also greater reflecting the additional time to travel along the corridor 304.

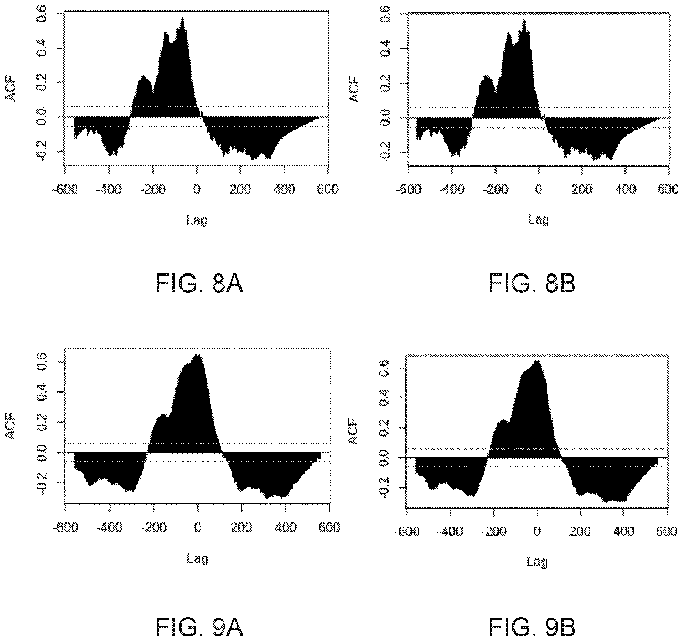

Figure 8A shows the cross-correlation between a first PM1 particulate level signal from the first PM sensor 308-a and a third PM1 particulate level signal from the third PM sensor 308-c. A correlation peak (max r = 0.580) is seen at a time lag of 65 epochs (325 seconds). This indicates that PM1 particles are travelling from the ward bay 306 into the corridor 304.

Figure 8B shows the cross-correlation between a first PM10 particulate level signal from the first PM sensor 308-a and a third PM10 particulate level signal from the third PM sensor 308-c. A correlation peak (max r = 0.573) is seen at a time lag of 65 epochs (325 seconds). This indicates that PM10 particles are travelling from the ward bay 306 into the corridor 304.

Figure 9A shows the cross-correlation between a second PM1 particulate level signal from the second PM sensor 308-b and a third PM1 particulate level signal from the third PM sensor 308-c. A correlation peak (max r = 0.649) is seen at a time lag of 6 epochs (30 seconds). This indicates that PM1 particles are travelling along the corridor 304 and rise and fall almost simultaneously at the second and third PM sensors 308-b, 308-c.

Figure 9B shows the cross-correlation between a second PM10 particulate level signal from the second PM sensor 308-b and a third PM10 particulate level signal from the third PM sensor 308-c. A correlation peak (max r = 0.648) is seen at a time lag of 6 epochs (30 seconds). This indicates that PM10 particles are travelling along the corridor 304 and rise and fall almost simultaneously at the second and third PM sensors 308-b, 308-c.

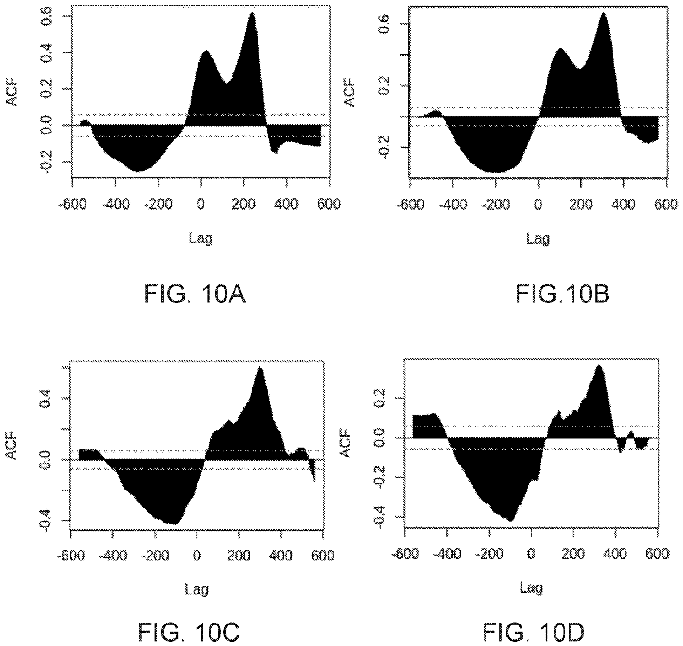

Figures 10A to 10D respectively show the cross-correlation between a first $CO_2$ level signal from a $CO_2$ sensor in the ward bay 306 and first, second, third and fourth PM1 particulate level signals corresponding to the first, second, third and fourth PM sensors 308-a, 308-b, 308-c, 308-d. The figures all show a correlation peak between the $CO_2$ level in the ward bay and the PM1 particulate level signal at each PM1 sensor. This is a complex relationship, which is difficult to interpret, but suggests that generally the $CO_2$ levels in the ward bay 306 rise before the PM1 levels (even in the ward bay 306).

No correlation was seen between $CO_2$ level signals 532-b, 532-c, 532-d in the corridor 304 and any other signals reinforcing that particulate matter flows cannot be tracked using $CO_2$ sensors alone. This is particularly the case when HVAC systems are present, which can dilute areas of concentrated very quickly.

The data of Figures 4 to 9d illustrate the tracking of particulate matter flow in an indoor setting following an aerosol event. The system 300 can respond by implementing an intervention measure as described above (e.g. closing automatic doors 322 before the particulate matter flow can reach that far or increasing the fan speed of an air purifier). Users can assess the data output from the system 300 to determine additional intervention measures or other remedial actions.

While the above discussion is primarily directed to systems in a medical setting, the disclosure is not limited thereto and it will be appreciated that the disclosed systems can be applied to any indoor setting including educational and commercial buildings.

It will be appreciated that any reference to "close to", "before", "shortly before", "after" "shortly after", "higher than", or "lower than", etc, can refer to the parameter in question being less than or greater than a threshold value, or between two threshold values, depending upon the context.

**Claims**

1. An air quality monitoring system (200) for monitoring aerosol flow in an indoor setting comprising:

   a plurality of particulate matter, PM, sensors (208), the plurality of PM sensors positioned at a corresponding plurality of positions in a monitoring area; and

   an air quality processing device (216) coupled to each of the plurality of PM sensors (208) via a communications network (218), the air quality processing device configured to:

   receive a particulate level signal from at least two of the plurality of PM sensors (208); and determine particulate matter flow between the at least two PM sensors (208) based on the corresponding particulate level signals.

2. The air quality monitoring system (200) of claim 1, wherein the air quality processing device (216) is configured to determine particulate matter flow between the at least two PM sensors (208) by:

   detecting an aerosol event at a first of the at least two PM sensors based on the particulate level signal exceeding a first event threshold; and

   detecting the aerosol event at a second of the at least two PM sensors based on the particulate level signal exceeding a second event threshold, wherein optionally the first event threshold comprises an adaptive event threshold.

3. The air quality monitoring system (200) of claim 2, wherein the second event threshold comprises a scaled value of the first event threshold.

4. The air quality monitoring system (200) of any preceding claim, wherein the air quality processing device (216) is configured to determine the particular matter flow based on a delay and / or amplitude difference between corresponding peaks of the particulate level signals.

5. The air quality monitoring system (200) of any preceding claim, wherein the air quality processing device (216) is configured to determine the particular matter flow by applying a cross-correlation to the particulate level signals associated with the at least two PM sensors (208).

6. The air quality monitoring system (200) of any preceding claim, wherein the air quality processing device (216) is configured to identify one or more of:

   a source of the particular matter flow;
   a path of the particular matter flow;
   a velocity of the particular matter flow;
   an attenuation of the particular matter flow; and / or
   one or more predicted destinations of the particular matter flow.

7. The air quality monitoring system (200) of any preceding claim, wherein the air quality processing device (216) is further configured to output an intervention signal configured to operate one or more air quality intervention mechanisms (222), wherein optionally the air quality intervention mechanisms comprise one or more of:

   an automatic door (222) or actuator thereof;
   operating parameters of an air filtering device (212);
   operating parameters of a heating, ventilation and air conditioning, HVAC, system; and
   an alert signal.

8. The air quality monitoring system (200) of claim 7, wherein the air quality processing device (216) is configured to output the intervention signal to operate one or more air quality intervention mechanisms (222) at:

   a location associated with the source of the particular matter;
   a location associated with the path of the particular matter flow; and / or
   a location associated with the one or more potential destinations of the particular matter flow.

9. The air quality monitoring system (200) of any preceding claim, wherein the air processing device (216) is further configured to:

analyse the particulate level signal for one or more PM sensors (208) over a time period to determine a particulate matter prevalence associated with the one or more PM sensors; and
output prevalence data indicating the particulate matter prevalence, wherein optionally the prevalence data indicates:

high risk regions of the monitoring area corresponding to one or more PM sensors with a particulate matter prevalence exceeding a first prevalence threshold; and / or
low risk regions of the monitoring area corresponding to one or more PM sensors with a particulate matter prevalence less than a second prevalence threshold.

10. The air quality monitoring system (200) of claim 9, wherein the particulate matter prevalence includes periodic aerosol events associated with the one or more PM sensors (208) and the prevalence data indicates:

the periodic aerosol events;
the times of occurrence of the periodic aerosol events; and / or
the location of the one or more PM sensors associated with the periodic aerosol event, wherein optionally the air processing device (216) is configured to output an intervention signal for operating one or more intervention mechanisms (222) at times corresponding to the periodic aerosol event.

11. The air quality monitoring system (200) of any preceding claim, wherein the air processing device (216) is configured to:

receive operational data relating to the monitoring area;
correlate one or more aerosol events with the operational data; and
identify aerosol event triggers based on the correlation.

12. The air quality monitoring system (200) of any preceding claim, wherein each of the PM sensors (208) is configured to measure a concentration of particulate matter in air with particle sizes in a range from a lower detection limit to a particulate matter rating of the PM sensor.

13. The air quality monitoring system (200) of any preceding claim, wherein two or more of the PM sensors (208) are positioned at different heights.

14. The air quality monitoring system (200) of any preceding claim, further comprising a plurality of further sensors, wherein the further sensors comprise one or more of:

a carbon dioxide ($CO_2$) sensor;
a humidity sensor;
a temperature sensor; and
a pressure sensor.

15. A method of monitoring particulate matter flow in a monitoring area of an indoor setting, the method comprising:
receiving a plurality of a particulate level signals from at least two particulate matter, PM, sensors (208) positioned in the monitoring area;
determining particulate matter flow between the at least two PM sensors (208) based on the corresponding particulate level signals.

**Patentansprüche**

1. Luftqualitätsüberwachungssystem (200) zum Überwachen des Aerosolflusses in einem Innenbereich, umfassend:

eine Vielzahl von Feinstaub-, PM-, Sensoren (208), wobei die Vielzahl von PM-Sensoren an einer entsprechenden Vielzahl von Positionen in einem Überwachungsbereich positioniert ist;
und

eine Luftqualitätsverarbeitungsvorrichtung (216), die über ein Kommunikationsnetzwerk (218) mit jedem der Vielzahl von PM-Sensoren (208) gekoppelt ist, wobei die Luftqualitätsverarbeitungsvorrichtung konfiguriert ist zum:

Empfangen eines Partikelniveausignals von mindestens zwei der Vielzahl von PM-Sensoren (208); und
Bestimmen des Feinstaubablaufs zwischen den mindestens zwei PM-Sensoren (208), basierend auf den entsprechenden Partikelniveausignalen.

2. Luftqualitätsüberwachungssystem (200) nach Anspruch 1, wobei die Luftqualitätsverarbeitungsvorrichtung (216) konfiguriert ist, den Feinstaubablauf zwischen den mindestens zwei PM-Sensoren (208) zu bestimmen, durch:

Erkennen eines Aerosolereignisses an einem ersten der mindestens zwei PM-Sensoren basierend auf dem Partikelniveausignal, das einen ersten Ereignisschwellenwert überschreitet; und
Erkennen des Aerosolereignisses an einem zweiten der mindestens zwei PM-Sensoren basierend auf dem Partikelniveausignal, das einen zweiten Ereignisschwellenwert überschreitet, wobei optional der erste Ereignisschwellenwert einen angepassten Ereignisschwellenwert umfasst.

3. Luftqualitätsüberwachungssystem (200) nach Anspruch 2, wobei der zweite Ereignisschwellenwert einen skalierten Wert des ersten Ereignisschwellenwerts umfasst.

4. Luftqualitätsüberwachungssystem (200) nach einem der vorstehenden Ansprüche, wobei die Luftqualitätsverarbeitungsvorrichtung (216) konfiguriert ist, den Feinstaubablauf basierend auf einer Verzögerung und/oder einer Amplitudendifferenz zwischen den entsprechenden Spitzen der Partikelniveausignale zu bestimmen.

5. Luftqualitätsüberwachungssystem (200) nach einem der vorstehenden Ansprüche, wobei die Luftqualitätsverarbeitungsvorrichtung (216) konfiguriert ist, den Feinstaubablauf durch Anwenden einer Kreuzkorrelation auf die Partikelniveausignale zu bestimmen, die den mindestens zwei PM-Sensoren (208) zugehörig sind.

6. Luftqualitätsüberwachungssystem (200) nach einem der vorstehenden Ansprüche, wobei die Luftqualitätsverarbeitungsvorrichtung (216) konfiguriert ist, eines oder mehrere der Folgenden zu identifizieren:

eine Quelle des Feinstaubablaufs;
einen Weg des Feinstaubablaufs;
eine Geschwindigkeit des Feinstaubablaufs;
eine Dämpfung des Feinstaubablaufs; und/oder
ein oder mehrere vorhergesagte Ziele des Feinstaubablaufs.

7. Luftqualitätsüberwachungssystem (200) nach einem der vorstehenden Ansprüche, wobei die Luftqualitätsverarbeitungsvorrichtung (216) ferner konfiguriert ist, ein Eingriffssignal auszugeben, das konfiguriert ist, einen oder mehrere Luftqualitätseingriffsmechanismen (222) zu betreiben, wobei die Luftqualitätseingriffsmechanismen optional eines oder mehrere der Folgenden umfassen:

eine automatische Tür (222) oder einen Aktuator davon;
Betriebsparameter einer Luftfiltervorrichtung (212);
Betriebsparameter eines Heizungs-, Belüftungs- und Klimatisierungs-, HVAC-, Systems; und
ein Alarmsignal.

8. Luftqualitätsüberwachungssystem (200) nach Anspruch 7, wobei die Luftqualitätsverarbeitungsvorrichtung (216) konfiguriert ist, das Eingriffssignal auszugeben, um einen oder mehrere Luftqualitätseingriffsmechanismen (222) zu betreiben an:

einer Stelle, die der Quelle des Feinstaubs zugehörig ist;
einer Stelle, die dem Weg des Feinstaubablaufs zugehörig ist; und/oder
einer Stelle, die dem einen oder mehreren potenziellen Zielen des Feinstaubablaufs zugehörig ist.

9. Luftqualitätsüberwachungssystem (200) nach einem der vorstehenden Ansprüche, wobei die Luftverarbeitungsvorrichtung (216) ferner konfiguriert ist zum:

Analysieren des Partikelniveausignals für einen oder mehrere PM-Sensoren (208) über einen Zeitraum, um eine Feinstaubprävalenz zu bestimmen, die dem einen oder den mehreren PM-Sensoren zugehörig ist; und Ausgeben der Prävalenzdaten, die auf die Feinstaubprävalenz hinweisen, wobei optional die Prävalenzdaten auf Folgendes hinweisen:

Hochrisikoregionen des Überwachungsbereichs, die einem oder mehreren PM-Sensoren mit einer Feinstaubprävalenz entsprechen, die einen ersten Prävalenzschwellenwert überschreitet; und/oder Regionen mit geringem Risiko des Überwachungsbereichs, die einem oder mehreren PM-Sensoren mit einer Feinstaubprävalenz entsprechen, die geringer als ein zweiter Prävalenzschwellenwert ist.

10. Luftqualitätsüberwachungssystem (200) nach Anspruch 9, wobei die Feinstaubprävalenz periodische Aerosolereignisse einschließt, die dem einen oder mehreren PM-Sensoren (208) zugehörig sind, und die Prävalenzdaten auf Folgendes hinweisen:

die periodischen Aerosolereignisse; die Zeiträume des Vorkommens der periodischen Aerosolereignisse; und/oder die Stelle des einen oder der mehreren PM-Sensoren, die dem periodischen Aerosolereignis zugehörig ist, wobei optional die Luftverarbeitungsvorrichtung (216) konfiguriert ist, ein Eingriffssignal zum Betreiben eines oder mehrerer Eingriffsmechanismen (222) zu Zeiten auszugeben, die dem periodischen Aerosolereignis entsprechen.

11. Luftqualitätsüberwachungssystem (200) nach einem der vorstehenden Ansprüche, wobei die Luftverarbeitungsvorrichtung (216) konfiguriert ist zum:

Empfangen der Betriebsdaten in Bezug auf den Überwachungsbereich; Korrelieren eines oder mehrerer Aerosolereignisse mit den Betriebsdaten; und Identifizieren der Aerosolereignisauslöser basierend auf der Korrelation.

12. Luftqualitätsüberwachungssystem (200) nach einem der vorstehenden Ansprüche, wobei jeder der PM-Sensoren (208) konfiguriert ist, eine Feinstaubkonzentration in der Luft mit Partikelgrößen in einem Bereich von einer niedrigeren Nachweisgrenze bis zu einer Feinstaubbewertung des PM-Sensors zu messen.

13. Luftqualitätsüberwachungssystem (200) nach einem der vorstehenden Ansprüche, wobei zwei oder mehrere der PM-Sensoren (208) in verschiedenen Höhen positioniert sind.

14. Luftqualitätsüberwachungssystem (200) nach einem der vorstehenden Ansprüche, ferner umfassend eine Vielzahl weiterer Sensoren, wobei die weiteren Sensoren eines oder mehrere der Folgenden umfassen:

einen Kohlendioxid- (CO2-) Sensor; einen Luftfeuchtigkeitssensor; einen Temperatursensor; und einen Drucksensor.

15. Verfahren zum Überwachen des Feinstaubablaufs in einem Überwachungsbereich in einem Innenraum, das Verfahren umfassend:

Empfangen einer Vielzahl von Partikelniveausignalen von mindestens zwei Feinstaub-, PM-, Sensoren (208), die in dem Überwachungsbereich positioniert sind; Bestimmen des Feinstaubablaufs zwischen den mindestens zwei PM-Sensoren (208), basierend auf den entsprechenden Partikelniveausignalen.

**Revendications**

1. Système de surveillance de la qualité de l'air (200) pour surveiller un flux d'aérosols dans un environnement intérieur comprenant :

une pluralité de capteurs de particules, PM (208), la pluralité de capteurs PM étant positionnée à une pluralité correspondante de positions dans une zone de surveillance ;
et
un dispositif de traitement de la qualité de l'air (216) couplé à chacun de la pluralité de capteurs de particules (208) via un réseau de communication (218), le dispositif de traitement de la qualité de l'air étant configuré pour :

recevoir un signal de niveau de particules provenant d'au moins deux de la pluralité de capteurs PM (208) ; et déterminer d'un flux de particules entre les au moins deux capteurs PM (208) sur la base des signaux de niveau de particules correspondants.

2. Système de surveillance de la qualité de l'air (200) selon la revendication 1, dans lequel le dispositif de traitement de la qualité de l'air (216) est configuré pour déterminer le flux de particules entre les au moins deux capteurs PM (208) en :

détectant un événement d'aérosol au niveau d'un premier des au moins deux capteurs PM sur la base du signal de niveau de particules dépassant un premier seuil d'événement ; et détectant l'événement d'aérosol au niveau d'un second des au moins deux capteurs PM sur la base du signal de niveau de particules dépassant un second seuil d'événement, le premier seuil d'événement comprenant éventuellement un seuil d'événement adaptatif.

3. Système de surveillance de la qualité de l'air (200) selon la revendication 2, dans lequel le second seuil d'événement comprend une valeur mise à l'échelle du premier seuil d'événement.

4. Système de surveillance de la qualité de l'air (200) selon une quelconque revendication précédente, dans lequel le dispositif de traitement de la qualité de l'air (216) est configuré pour déterminer le flux de particules sur la base d'une temporisation et/ou d'une différence d'amplitude entre les pics correspondants des signaux de niveau de particules.

5. Système de surveillance de la qualité de l'air (200) selon une quelconque revendication précédente, dans lequel le dispositif de traitement de la qualité de l'air (216) est configuré pour déterminer le flux de particules en appliquant une corrélation croisée aux signaux de niveau de particules associés aux au moins deux capteurs PM (208).

6. Système de surveillance de la qualité de l'air (200) selon une quelconque revendication précédente, dans lequel le dispositif de traitement de la qualité de l'air (216) est configuré pour identifier un ou plusieurs des éléments suivants :

une source de flux de particules ;
un chemin du flux de particules ;
une vitesse du flux de particules ;
une atténuation du flux de particules ; et/ou
une ou plusieurs destinations prévues du flux de particules.

7. Système de surveillance de la qualité de l'air (200) selon une quelconque revendication précédente, dans lequel le dispositif de traitement de la qualité de l'air (216) est en outre configuré pour émettre un signal d'intervention configuré pour actionner un ou plusieurs mécanismes d'intervention sur la qualité de l'air (222), les mécanismes d'intervention sur la qualité de l'air comprenant éventuellement un ou plusieurs des éléments suivants :

une porte automatique (222) ou son actionneur ;
des paramètres de fonctionnement d'un dispositif de filtration d'air (212) ;
des paramètres de fonctionnement d'un système de chauffage, de ventilation et de climatisation (CVC) ; et
un signal d'alerte.

8. Système de surveillance de la qualité de l'air (200) selon la revendication 7, dans lequel le dispositif de traitement de la qualité de l'air (216) est configuré pour émettre le signal d'intervention afin de faire fonctionner un ou plusieurs mécanismes d'intervention sur la qualité de l'air (222) au niveau :

d'un emplacement associé à la source des particules ;
d'un emplacement associé au trajet du flux de particules ; et/ou
d'un emplacement associé à une ou plusieurs destinations potentielles du flux de particules.

**9.** Système de surveillance de la qualité de l'air (200) selon une quelconque revendication précédente, dans lequel le dispositif de traitement de l'air (216) est en outre configuré pour :

analyser le signal de niveau de particules pour un ou plusieurs capteurs PM (208) sur une période de temps pour déterminer une prévalence de particules associée aux un ou plusieurs capteurs PM ; et
émettre des données de prévalence indiquant la prévalence des particules, dans lequel éventuellement les données de prévalence indiquent :

des régions à haut risque de la zone de surveillance correspondant à un ou plusieurs capteurs PM avec une prévalence de particules dépassant un premier seuil de prévalence ; et/ou
des régions à faible risque de la zone de surveillance correspondant à un ou plusieurs capteurs PM avec une prévalence de particules inférieure à un second seuil de prévalence.

**10.** Système de surveillance de la qualité de l'air (200) selon la revendication 9, dans lequel la prévalence de particules comporte des événements d'aérosols périodiques associés aux un ou plusieurs capteurs PM (208) et les données de prévalence indiquent :

les événements périodiques d'aérosols ;
les heures d'occurrence des événements périodiques d'aérosols ; et/ou
l'emplacement des un ou plusieurs capteurs PM associés à l'événement périodique d'aérosol, dans lequel éventuellement le dispositif de traitement de l'air (216) est configuré pour émettre un signal d'intervention afin de faire fonctionner un ou plusieurs mécanismes d'intervention (222) à des moments correspondant à l'événement périodique d'aérosol.

**11.** Système de surveillance de la qualité de l'air (200) selon une quelconque revendication précédente, dans lequel le dispositif de traitement de l'air (216) est configuré pour :

recevoir des données opérationnelles relatives à la zone de surveillance ;
corréler un ou plusieurs événements d'aérosol avec les données opérationnelles ; et
identifier des déclencheurs d'événements d'aérosols en fonction de la corrélation.

**12.** Système de surveillance de la qualité de l'air (200) selon une quelconque revendication précédente, dans lequel chacun des capteurs PM (208) est configuré pour mesurer une concentration de particules dans l'air avec des tailles de particules dans une plage allant d'une limite de détection inférieure à une évaluation des particules du capteur PM.

**13.** Système de surveillance de la qualité de l'air (200) selon une quelconque revendication précédente, dans lequel deux capteurs PM (208) ou plus sont positionnés à différentes hauteurs.

**14.** Système de surveillance de la qualité de l'air (200) selon une quelconque revendication précédente, comprenant en outre une pluralité de capteurs supplémentaires, les capteurs supplémentaires comprenant un ou plusieurs des éléments suivants :

un capteur de dioxyde de carbone (CO2) ;
un capteur d'humidité ;
un capteur de température ; et
un capteur de pression.

**15.** Procédé de surveillance d'un flux de particules dans une zone de surveillance d'un environnement intérieur, le procédé comprenant :

la réception d'une pluralité de signaux de niveau de particules provenant d'au moins deux capteurs de particules, PM (208), positionnés dans la zone de surveillance ;
la détermination d'un flux de particules entre les au moins deux capteurs PM (208) sur la base des signaux de niveau de particules correspondants.

FIG. 1

FIG. 2

FIG. 3

FIG. 4

FIG. 5

FIG. 6A

FIG. 6B

FIG. 7A

FIG. 7B

FIG. 8A

FIG. 8B

FIG. 9A

FIG. 9B

FIG. 10A

FIG.10B

FIG. 10C

FIG. 10D

**REFERENCES CITED IN THE DESCRIPTION**

**Patent documents cited in the description**

- WO 2020146315 A